# EUROPEAN PATENT APPLICATION

(11) **EP 4 527 385 A1**
(43) Date of publication of application: **26.03.2025**
(21) Application number: 23807554.3
(22) Date of filing: 12.05.2023
(51) Int. Cl.: A61K 31/41, A61K 45/06, A61P 7/10, A61P 9/00, A61P 9/04, A61P 9/12, A61P 11/00, A61P 29/00, A61P 43/00

(54) **IL-6 AND/OR IL-1? INHIBITOR**

(30) Priority: 16.05.2022 JP 2022080531
(71) Applicant: AGC Inc., Chiyoda-ku Tokyo 100-8405 (JP); Kyushu University, National University Corporation, Nishi-ku Fukuoka-shi Fukuoka 819-0395 (JP)
(72) Inventor: MATSUMURA, Yasushi, Tokyo 100-8405 (JP); ABE, Kohtaro, Fukuoka-shi, Fukuoka 819-0395 (JP); KIMURO, Keiji, Fukuoka-shi, Fukuoka 819-0395 (JP); IMABAYASHI, Misaki, Fukuoka-shi, Fukuoka 819-0395 (JP)
(74) Representative: Müller-Boré & Partner Patentanwälte PartG mbB
(86) International application number: PCT/JP2023/017859
(87) International publication number: WO 2023/223951

(57) **Abstract**

The present invention aims to provide a medicament for treating circulatory diseases accompanied by inflammation of a cardiovascular system related to IL-6 and/or IL-1β. The present invention relates to an IL-6 and/or IL-1β suppressor consisting of a compound represented by the following formula (I) : 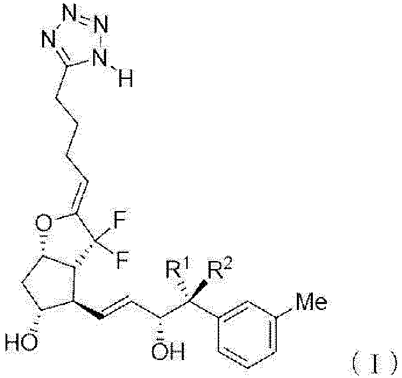 wherein each symbol is as described in the DESCRIPTION, or a pharmaceutically acceptable salt thereof. The present invention also relates to a medicament for treating circulatory diseases accompanied by inflammation of a cardiovascular system related to IL-6 and/or IL-1β, which medicament contains the aforementioned compound or a pharmaceutically acceptable salt thereof as an active ingredient.

## Description

### [Technical Field]

The present invention relates to a prostaglandin derivative that suppresses excessive gene expression and production of IL-6 and/or IL-1β, and is useful for the treatment of circulatory diseases associated with inflammation of a cardiovascular system related to IL-6 and/or IL-1β.

### [Background Art]

Interleukin-6 (IL-6) and interleukin-1β (IL-1β) are cytokines with diverse physiological actions and play important roles in regulating immune responses and inflammatory responses. However, excessive gene expression and continued production thereof cause various pathological conditions.

For example, it has been pointed out that immune cells and the cytokines they produce are involved in the development of heart failure. It is known that IL-6 in the blood increases in patients with heart failure or myocarditis, and is also related to prognosis (Non Patent Literatures 1, 2).

It has been reported that inflammatory cytokines such as IL-6 and the like are elevated in the blood of patients with pulmonary hypertension, and become a poor prognostic factor (Non Patent Literature 3). It has also been reported that, in patients with pulmonary hypertension, cell membrane-bound IL-6 receptors are highly expressed in pulmonary arterial vascular smooth muscle cells, and IL-6 signaling provides an anti-apoptotic resistance to vascular smooth muscle cells, thus contributing to the remodeling of pulmonary artery (Non Patent Literature 4).

It has been confirmed that IL-6 is overexpressed in patients with systemic scleroderma, that IL-6 strongly affects fibrosis, and that IL-6 inhibitors show therapeutic effects against interstitial lung diseases associated with systemic scleroderma (Non Patent Literature 5).

Regarding pulmonary arterial hypertension, it has been confirmed that blood IL-6 concentration is high particularly in patients with pulmonary arterial hypertension associated with connective tissue disease or portal hypertension, and it has been reported that patients with higher blood IL-6 concentrations have poorer prognosis (Non Patent Literature 6).

It was reported that serum IL-6 concentration increased in correlation with disease activity in patients with Takayasu's arteritis, and later, IL-6 inhibitors were shown to be effective and have been used in clinical practice (Non Patent Literatures 7, 8, 9).

On the other hand, IL-1β is deeply involved in inflammatory responses and also plays an important role in controlling metabolic cardiac hypertrophy. Given the favorable clinical results for cardiovascular events and heart failure obtained in clinical trials targeting IL-1β, the control of chronic inflammation with IL-1β inhibitors has attracted attention in recent years as a new treatment method for heart failure (Non Patent Literatures 10, 11).

In light of the above, a medicament that suppresses excessive gene expression and production of IL-6 and/or IL-1β (hereinafter at times referred to as "IL-6 and/or IL-1β suppressor") is expected as a therapeutic agent for circulatory diseases associated with inflammation of a cardiovascular system.

Cardiac hypertrophy refers to a condition in which the heart muscle is hypertrophied to increase the myocardial weight. Specifically, it is a phenomenon that occurs when the load on the heart increases for some reason, and cardiac hypertrophy occurs as a compensatory mechanism. Hypertrophy due to thickening of the heart wall is often caused by hypertension, valvular disease, cardiomyopathy, and the like, and can also be caused by myocardial abnormality associated with hyperthyroidism or hypothyroidism, muscular dystrophy, and the like. Cardiac hypertrophy is a risk factor that causes heart failure and coronary artery disease, and a treatment to suppress or regress cardiac hypertrophy is important.

Heart failure is defined as a clinical syndrome in which some heart dysfunction, i.e., structural and/or functional abnormality in the heart, collapses the compensatory mechanism of cardiac pump function, resulting in the emergence of breathing difficulty, fatigue and edema, along with which exercise tolerance decreases. For example, in acute myocarditis, inflammatory cell infiltration and myocardial edema cause cardiac hypertrophy, which leads to heart failure. In heart failure, ventricular hypertrophy, enlargement, systolic dysfunction, diastolic dysfunction, and the like are known to progress, which is called myocardial remodeling. This process is accompanied by myocardial wall hypertrophy, cardiomyocyte hypertrophy, degeneration, and interstitial fibrosis. Improvement of myocardial remodeling is considered one of the important goals in the treatment of heart failure, and a therapeutic drug with new action mechanism has been desired.

Right heart failure is a condition in which the function of the right ventricular system is impaired during the process in which venous blood that has returned from the whole body to the heart is transported to the lungs through the right ventricular system, and is a life-threatening disease if left untreated. Right ventricular hypertrophy is one of the main changes in right heart failure. The main cause of right heart failure is pulmonary circulatory disorder, and in particular, the pulmonary circulation is affected by pulmonary embolism or pulmonary hypertension.

Pulmonary embolism is generally accompanied by characteristic clinical signs, such as acute shortness of breath, collapse-like symptoms, and chest pain. Pulmonary embolism is often caused by thrombus that has occurred in the veins of the lower extremities for some reason, entered into pulmonary artery through blood vessels, and occluded the pulmonary artery.

Pulmonary hypertension is a refractory disease accompanied by elevation of pulmonary artery pressure, with the background of pulmonary vascular remodeling caused by pulmonary arterial endothelial disorder, smooth muscle proliferation, inflammation and immune abnormality, and the like.

Pulmonary vascular remodeling occurs mainly due to proliferation of arterial endothelial cells and smooth muscle cells in patients, and as symptoms progress, muscularization of pulmonary arterioles, thickening of the media, thickening of the intima, stenosis of the vascular space due to proliferation of endothelial cells, plexiform lesions, and the like are observed. As mentioned above, it has been clarified that IL-6 is elevated in the serum and lungs of patients, indicating the involvement of inflammatory mechanisms in pulmonary vascular remodeling.

The current clinical classification of pulmonary hypertension includes five groups based on the 2013 Nice classification (Non Patent Literature 12).

Group 1: pulmonary arterial hypertension (PAH);
   1 idiopathic PAH
   2 hereditary PAH
   3 drug- and toxin-induced PAH
   4 PAH associated with various diseases
      4.1 connective tissue disease
      4.2 HIV infectious disease
      4.3 portal hypertension
      4.4 congenital cardiac disease
      4.5 schistosomiasis
Group 1': pulmonary veno-occlusive disease (PVOD) and/or lung pulmonary capillary hemangiomatosis (PCH);
Group 1'': persistent pulmonary hypertension of the newborn (PPHN)
Group 2: pulmonary hypertension resulting from left heart disease;
Group 3: pulmonary hypertension resulting from pulmonary disease and/or hypoxia;
Group 4: chronic thromboembolic pulmonary hypertension (CTEPH) ;
Group 5: pulmonary hypertension with unknown multifactorial mechanism.

Pulmonary arterial hypertension (PAH), which is Group 1 of pulmonary hypertension, is characterized by prominent vasoconstriction and abnormal proliferation of cells in the pulmonary artery wall. This abnormal proliferation causes severe vasoconstriction of blood vessels in the lungs, leading to a marked increase in pulmonary vascular resistance (PVR) and a sustained rise in pulmonary artery pressure (PAP). As a result, right heart failure is developed and the patient has a poor prognosis. Pulmonary hypertension accompanied by right heart failure is highly severe, and if left untreated, the average life expectancy in the case of PAH is said to be 2.8 years from the time of diagnosis.

In PAH associated with connective tissue disease, immunosuppressive therapy with steroids or cyclophosphamide is effective for some types of PAH associated with connective tissue disease. However, the prognosis of PAH associated with mixed connective tissue disease or systemic scleroderma is poor, and the prognosis of PAH associated with systemic scleroderma is particularly poor.

There is no therapeutic drug with clear clinical evidence for PAH associated with portal hypertension.

In Group 2 of pulmonary hypertension, posterior capillary pulmonary hypertension occurs due to various left ventricular diseases such as left ventricular systolic dysfunction, diastolic failure, valvular disease, and the like. In some cases, remodeling of pulmonary artery is also involved, the prognosis is worse than that of PAH, and it is also a factor that worsens the prognosis of heart failure. As a treatment of the pulmonary hypertension Group 2, clinical trials of vasodilators have been conducted; however, a prognosis improvement effect has not been achieved.

Group 3 of pulmonary hypertension is associated with chronic lung diseases such as chronic obstructive pulmonary diseases (COPD), interstitial lung disease, and the like, and hypoxia. It is difficult to administer vasodilators to patients without causing deterioration of gas exchange function, and there are currently no approved therapeutic drugs for patients with Group 3 pulmonary hypertension.

Chronic thromboembolic pulmonary hypertension (CTEPH), which is Group 4 of pulmonary hypertension, is a disease caused by organized thrombus in the pulmonary artery. As a treatment of CTEPH, surgical procedures and balloon pulmonary angioplasty are performed, and specific vasodilators have also been used. As therapeutic drugs for pulmonary hypertension, three lines of vasodilators are known: prostacyclin derivatives (epoprostenol, beraprost, treprostinil, selexipag, etc.) belonging to the prostacyclin pathway, endothelin receptor antagonists (bosentan, ambrisentan, etc.) belonging to the endothelin pathway, and phosphodiesterase 5 inhibitors (sildenafil, tadalafil, etc.) and guanylate cyclase stimulators (riociguat, etc.) of NO-based preparations. These vasodilators are used for Group 1 PAH, and some are also used for Group 4 CTEPH. Even though the prognosis of PAH has been improved by combination therapy with these vasodilators, it is still not sufficient and CTEPH is still a disease with a poor prognosis. In particular, PAH associated with connective tissue diseases such as PAH associated with systemic scleroderma is accompanied by remodeling of not only the pulmonary artery but also the pulmonary vein, and there are not a small number of patients for whom conventional vasodilators are not sufficiently effective or who develop fatal complications such as pulmonary edema due to pulmonary vein stenosis.

Prostacyclin derivatives bind to prostacyclin receptors (IP receptors) to exert vasodilating effects, but the presence of nonresponders whose effects are insufficient has posed a problem. It is also considered that one of the causes thereof is the reduced expression of IP receptors in patients with pulmonary hypertension (Non Patent Literatures 13, 14). In addition, the use of prostacyclin derivatives is limited due to various side effects such as shock, flushing, hypotension, headache, jaw pain, nausea·vomiting, diarrhea, bleeding and the like.

As described above, the current primary method of treating pulmonary hypertension is the administration of vasodilators, and in order to avoid fatal complications such as pulmonary edema, a therapeutic drug that does not cause acute vasodilation is needed. In order to effectively treat serious diseases accompanied by pulmonary vascular remodeling, such as pulmonary hypertension, the development of a new therapeutic drug by an action mechanism different from that of conventional treatment methods that use vasodilators and the like has been demanded.

Large vessel vasculitis includes Takayasu's arteritis and giant cell arteritis, and inflammation of blood vessels causes serious complications such as aortostenosis, aortic aneurysm, aortic dissection, pulmonary stenosis, heart failure, renal failure, pulmonary hypertension, ophthalmic artery lesion and the like. Takayasu's arteritis is characterized by necrosis of vascular smooth muscle cell, destruction of elastic fibers, fibrosis, and inflammatory thickening of the adventitia. In giant cell arteritis, vascular lesions are observed such as the appearance of giant cells in the medial and intimal, and occlusion of the lumen due to thickening of the intima. IL-6 inhibitors show a remodeling improving effect on these vascular lesions, and are used as therapeutic drugs therefor (Non Patent Literature 15).

The present inventors found that a compound represented by the following formula (1): 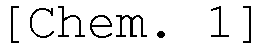 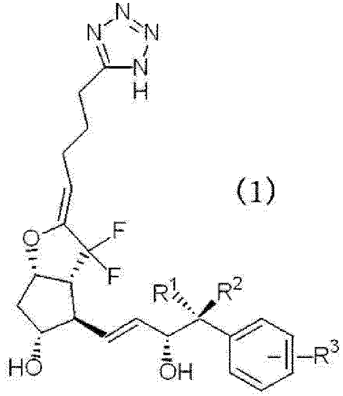 wherein R¹ and R² are each independently a hydrogen atom or a linear alkyl group having 1 to 3 carbon atoms, and R³ is a hydrogen atom, an alkyl group having 1 - 4 carbon atoms, an alkoxyalkyl group, an aryl group, a halogen atom, or a halogenated alkyl group) or a pharmaceutically acceptable salt thereof shows a therapeutic effect on ulcerative colitis and the like (Patent Literatures 1, 2, and Non Patent Literatures 16, 17). The pharmacological action of the compound against osteoarthritis of the knee has also been reported (Non Patent Literature 18). However, it is not known that the compound represented by the aforementioned formula (1) exhibits a suppressive action against excessive gene expression or production of IL-6 and/or IL-1β, and therefore, there is no report on the therapeutic effect for circulatory diseases accompanied by inflammation of a cardiovascular system related to IL-6 and/or IL-1β.

### [Citation List]

### [Patent Literature]

[Patent Literature 1]
   WO 2010/029925
[Patent Literature 2]
   WO 2011/111714

### [Non Patent Literature]

[Non Patent Literature 1]
   J. Am. Coll. Cardiol., 1998, 31, 391-398
[Non Patent Literature 2]
   J. Cardiology, 2021, 78, 157-165
[Non Patent Literature 3]
   Circulation, 2010, 122, 920-927
[Non Patent Literature 4]
   J. Clin. Invest., 2018, 128, 1956-1970
[Non Patent Literature 5]
   Arthritis Rheumatol., 2022, 74, 13-27
[Non Patent Literature 6]
   Eur. Resp. J., 2020, 55, 1901761
[Non Patent Literature 7]
   Circulation, 1999, 100, 55-60
[Non Patent Literature 8]
   Reumatology, 2006, 45, 545-548
[Non Patent Literature 9]
   Ann. Reum. Dis., 2018, 77, 348-354
[Non Patent Literature 10]
   N. Engl. J. Med., 2017, 377, 1119-1131
[Non Patent Literature 11]
   Circ. Heart Fail., 2018, 11, e005036
[Non Patent Literature 12]
   Guidelines for Treatment of Pulmonary Hypertension (JCS 2017/JPCPHS 2017), P.8-11
[Non Patent Literature 13]
   Am. J. Respir. Crit. Care Med., 2010, 182, 1161-1170
[Non Patent Literature 14]
   Am. J. Respir. Crit. Care Med., 2001, 164, 314-318
[Non Patent Literature 15]
   Guidelines for Management of Vasculitis Syndrome (JCS 2017), P.9-39
[Non Patent Literature 16]
   Eur. J. Pharmacol., 2015, 754, 179-189
[Non Patent Literature 17]
   J. Gastroenterol., 2019, 54, 608-620
[Non Patent Literature 18]
   Sci. Rep., 2019, 20329-20341

### [Summary of Invention]

### [Technical Problem]

The present invention aims to provide a medicament for treating circulatory diseases accompanied by inflammation of a cardiovascular system related to IL-6 and/or IL-1β, by suppressing excessive gene expression or production of IL-6 and/or IL-1β.

### [Solution to Problem]

The present inventors have conducted intensive studies in an attempt to solve the above-mentioned problems and found that a compound represented by the following formula (I): 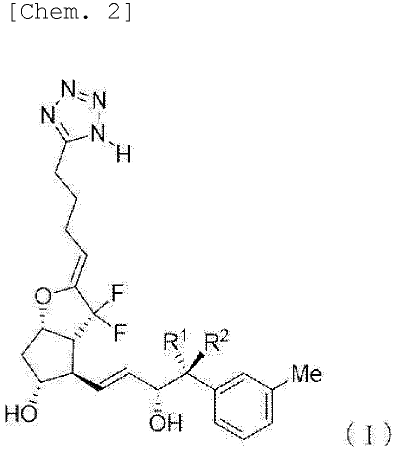 wherein R¹ and R² are each independently a hydrogen atom or a methyl group (hereinafter at times referred to as "the compound of the present invention") or a pharmaceutically acceptable salt thereof is a superior IL-6 and/or IL-1β suppressor, and that a medicament containing the IL-6 and/or IL-1β suppressor as an active ingredient is useful for the treatment of circulatory diseases accompanied by inflammation of a cardiovascular system related to IL-6 and/or IL-1β, and completed the present invention.

That is, the present invention provides the following.
[1] An IL-6 and/or IL-1β suppressor consisting of a compound represented by the formula (I): 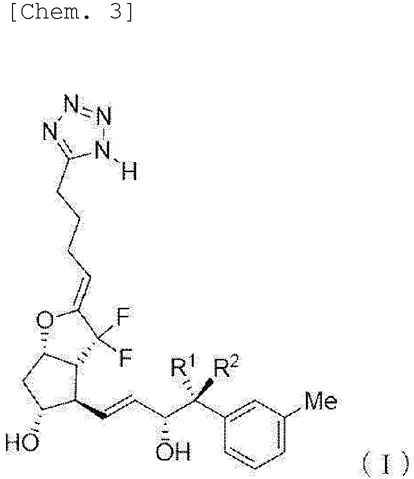 wherein R¹ and R² are each independently a hydrogen atom or a methyl group, or a pharmaceutically acceptable salt thereof.
[2] The suppressor of the above-mentioned [1], wherein the aforementioned compound represented by the formula (I) is 4-[(Z)-(1S,5R,6R,7R)-6-[(1E,3R,4R)-3-hydroxy-4-(m-tolyl)-1-pentenyl]-7-hydroxy-2-oxa-4,4-difluoro-bicyclo[3.3.0]octan-3-ylidene]-1-(tetrazol-5-yl)butane.
[3] The suppressor of the above-mentioned [1] or [2], which is an IL-6 and IL-1β suppressor.
[4] A medicament for the prophylaxis and/or treatment of a circulatory disease accompanied by inflammation of a cardiovascular system related to IL-6 and/or IL-1β, comprising the suppressor of any of the above-mentioned [1] to [3] as an active ingredient (hereinafter at times referred to as "the medicament of the present invention").
[5] The medicament of the above-mentioned [4], wherein the cardiovascular system is a pulmonary blood vessel.
[6] The medicament of the above-mentioned [4], wherein the cardiovascular system is the myocardium.
[7] The medicament of any of the above-mentioned [4] to [6], wherein the circulatory disease is selected from the group consisting of cardiac hypertrophy, heart failure, myocarditis, and cardiomyopathy.
[8] The medicament of the above-mentioned [4], wherein the circulatory disease is cardiac hypertrophy.
[9] The medicament of the above-mentioned [4], wherein the circulatory disease is heart failure.
[10] The medicament of the above-mentioned [9], wherein the heart failure is right heart failure.
[11] The medicament of the above-mentioned [4], wherein the circulatory disease is pulmonary hypertension accompanied by right heart failure.
[12] The medicament of the above-mentioned [4], wherein the circulatory disease is pulmonary hypertension associated with a left heart disease.
[13] The medicament of the above-mentioned [4], wherein the circulatory disease is pulmonary hypertension associated with lung disease and/or hypoxemia.
[14] The medicament of the above-mentioned [4], wherein the circulatory disease is chronic thromboembolic pulmonary hypertension.
[15] The medicament of the above-mentioned [4], wherein the circulatory disease is pulmonary arterial hypertension associated with a connective tissue disease.
[16] The medicament of the above-mentioned [15], wherein the connective tissue disease is a mixed connective tissue disease or systemic scleroderma.
[17] The medicament of the above-mentioned [4], wherein the circulatory diseases is pulmonary arterial hypertension associated with portal hypertension.
[18] The medicament of the above-mentioned [4], wherein the circulatory disease is selected from the group consisting of large vessel vasculitis, aortic aneurysm, and artery dissociation.
[19] The medicament of any of the above-mentioned [4] to [18], which is for oral administration.
[20] The medicament of the above-mentioned [19], which is orally administered in two or more divided doses per day.
[21] The medicament of any of the above-mentioned [4] to [20], which is administered in combination with one or two or more kinds of other drugs.
[22] The medicament of the above-mentioned [21], wherein the medicament of the above-mentioned [4] and one or two or more kinds of other drugs are contained separately as active ingredients of different preparations and are administered simultaneously or at different times.
[23] The medicament of the above-mentioned [21], wherein the medicament of the above-mentioned [4] and one or two or more kinds of other drugs are contained in a single preparation.
[24] The medicament of any of the above-mentioned [21] to [23], wherein the other drug is a vasodilator.
[25] A pharmaceutical composition comprising the suppressor of any of the above-mentioned [1] to [3], and a pharmaceutically acceptable carrier (hereinafter at times referred to as "the pharmaceutical composition of the present invention").
[26] A cardiovascular remodeling suppressor comprising the suppressor of any of the above-mentioned [1] to [3] as an active ingredient.
[27] A pulmonary artery muscularization suppressor comprising the suppressor of any of the above-mentioned [1] to [3] as an active ingredient.
[28] A compound represented by the formula (I): 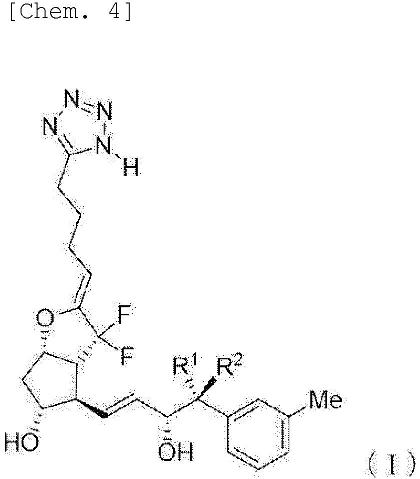 wherein R¹ and R² are each independently a hydrogen atom or a methyl group, or a pharmaceutically acceptable salt thereof for use in the prophylaxis and/or treatment of a circulatory disease accompanied by inflammation of a cardiovascular system related to IL-6 and/or IL-1β.
[29] The compound of the above-mentioned [28], wherein the circulatory disease accompanied by inflammation of a cardiovascular system related to IL-6 and/or IL-1β is a disease selected from the group consisting of cardiac hypertrophy, heart failure, myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease, pulmonary arterial hypertension associated with portal hypertension, large vessel vasculitis, aortic aneurysm, and artery dissociation, or a pharmaceutically acceptable salt thereof.
[30] Use of a compound represented by the formula (I): 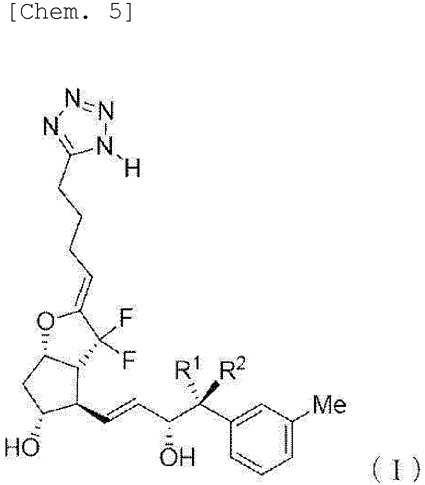 wherein R¹ and R² are each independently a hydrogen atom or a methyl group, or a pharmaceutically acceptable salt thereof for the production of a prophylactic and/or therapeutic agent for a circulatory disease accompanied by inflammation of a cardiovascular system related to IL-6 and/or IL-1β.
[31] The use of the above-mentioned [30], wherein the circulatory disease accompanied by inflammation of a cardiovascular system related to IL-6 and/or IL-1β is a disease selected from the group consisting of cardiac hypertrophy, heart failure, myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease, pulmonary arterial hypertension associated with portal hypertension, large vessel vasculitis, aortic aneurysm, and artery dissociation.
[32] A method for preventing and/or treating a circulatory disease accompanied by inflammation of a cardiovascular system related to IL-6 and/or IL-1β in a mammal, comprising administering a pharmaceutically effective amount of a compound represented by the formula (I): 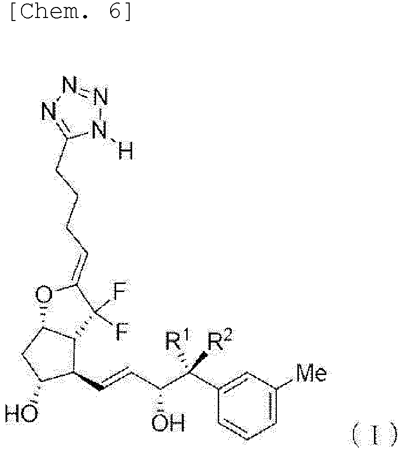 wherein R¹ and R² are each independently a hydrogen atom or a methyl group, or a pharmaceutically acceptable salt thereof, to the mammal.
[33] The method of the above-mentioned [32], wherein the circulatory disease accompanied by inflammation of a cardiovascular system related to IL-6 and/or IL-1β is a disease selected from the group consisting of cardiac hypertrophy, heart failure, myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease, pulmonary arterial hypertension associated with portal hypertension, large vessel vasculitis, aortic aneurysm, and artery dissociation.

### [Advantageous Effects of Invention]

The compound of the present invention or a pharmaceutically acceptable salt thereof is useful as an IL-6 and/or IL-1β suppressor. According to the present invention, moreover, a medicament for the prophylaxis and/or treatment of circulatory diseases accompanied by inflammation of a cardiovascular system related to IL-6 and/or IL-1R, in particular, a disease selected from the group consisting of cardiac hypertrophy (e.g., right ventricular hypertrophy), heart failure (e.g., right heart failure), myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease (e.g., mixed connective tissue disease, systemic scleroderma), pulmonary arterial hypertension associated with portal hypertension, large vessel vasculitis (e.g., Takayasu's arteritis, giant cell arteritis), aortic aneurysm, and artery dissociation, which contains, as an active ingredient, an IL-6 and/or IL-1β suppressor consisting of the compound of the present invention or a pharmaceutically acceptable salt thereof, can be provided.

### [Brief Description of Drawings]

[Fig. 1]

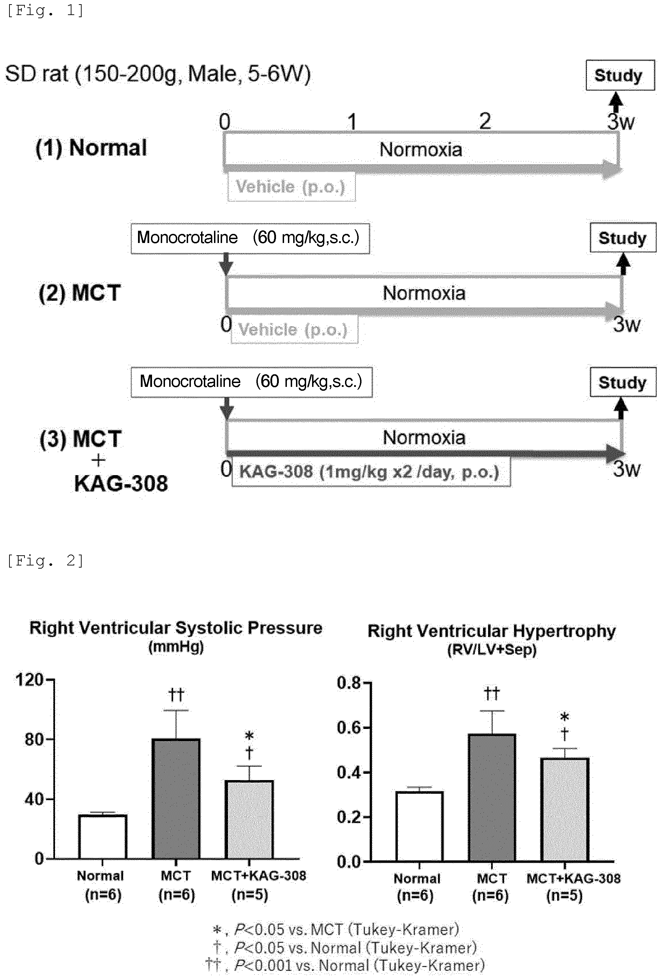

   Fig. 1 shows an administration method for each group.
[Fig. 2]
   Fig. 2 shows the effect of KAG-308 (2 mg/kg/day) on rats with monocrotaline-induced pulmonary hypertension at 3 weeks after administration. The left graph shows the effect on right ventricular systolic pressure, and the right graph shows the effect on right ventricular hypertrophy.
[Fig. 3]

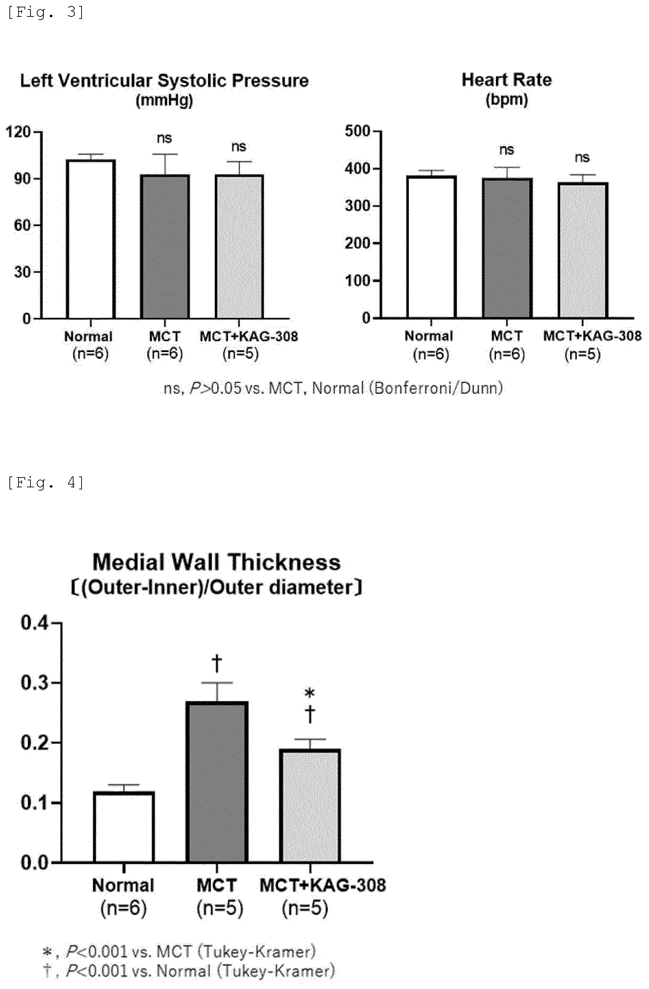

   Fig. 3 shows the effect of KAG-308 (2 mg/kg/day) on rats with monocrotaline-induced pulmonary hypertension at 3 weeks after administration. The left graph shows the effect on left ventricular systolic pressure, and the right graph shows the effect on heart rate.
[Fig. 4]
   Fig. 4 shows the suppressive effect of KAG-308 (2 mg/kg/day) on medial wall thickness of pulmonary artery as determined by pulmonary histopathological analysis in rats with monocrotaline-induced pulmonary hypertension at 3 weeks after administration.
[Fig. 5]

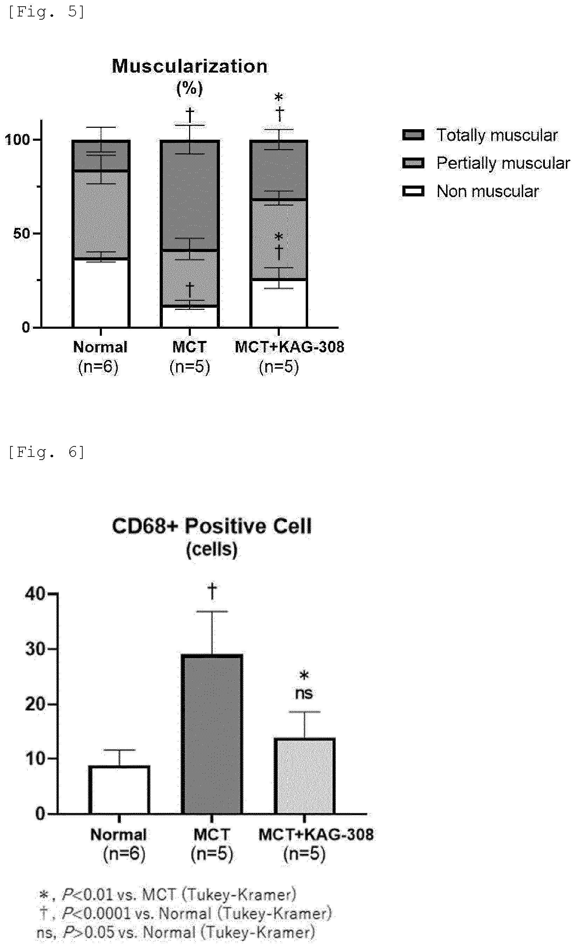

   Fig. 5 shows the suppressive effect of KAG-308 (2 mg/kg/day) on muscularization of small pulmonary artery as determined by pulmonary histopathological analysis in rats with monocrotaline-induced pulmonary hypertension at 3 weeks after administration.
[Fig. 6]
   Fig. 6 shows the suppressive effect of KAG-308 (2 mg/kg/day) on infiltration of inflammatory macrophage as determined by pulmonary histopathological analysis in rats with monocrotaline-induced pulmonary hypertension at 3 weeks after administration.
[Fig. 7]

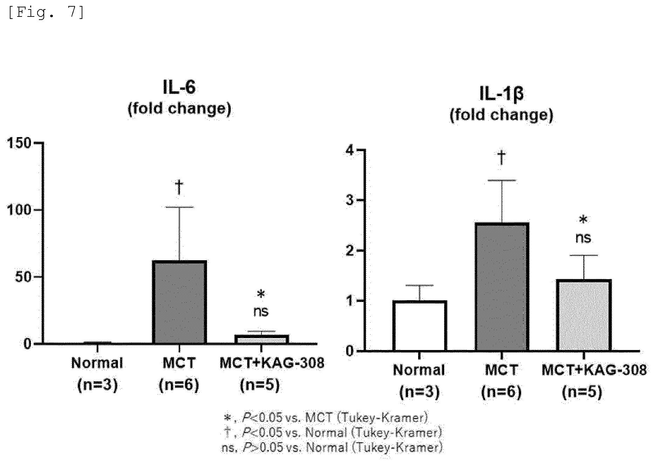

   Fig. 7 shows the quantitative RT-PCR analysis results at 3 weeks after administration of KAG-308 (2 mg/kg/day) to rats with monocrotaline-induced pulmonary hypertension. The left graph shows the mRNA expression level of IL-6, and the right graph shows the mRNA expression level of IL-1β.

### [Description of Embodiments]

The definitions of the terms and respective symbols used in the present specification are explained below. Unless otherwise defined, all technical and scientific terms used in the present specification have the same meaning as generally understood by one of ordinary skill in the technique field to which the present invention belongs.

In the present specification, the "pharmaceutically acceptable salt" means a salt of tetrazole group moiety of the compound of the present invention and a non-toxic base acceptable as a medicament, including inorganic base and organic base, and is a compound in which the hydrogen atom of the tetrazole group is substituted by a cation. Examples of the cation include alkali metal cations such as Na⁺, K⁺ and the like, metal cations other than alkali metals such as 1/2 Ca²⁺, 1/2 Mg²⁺, 1/2 Zn²⁺, 1/3 Al³⁺ and the like, NH₄⁺, and ammonium cations of organic amines and amino acids such as triethanolamine, diethanolamine, ethanolamine, tromethamine, lysine, arginine and the like. Preferred cations are sodium ion and potassium ion.

Specific examples of the salt derived from a pharmaceutically acceptable non-toxic inorganic base include the aforementioned sodium salt, potassium salt, calcium salt, magnesium salt, zinc salt, aluminum salt, ammonium salt and the like, as well as salts such as lithium salt, copper salt, ferric salt, ferrous salt, manganic salt, manganese salt and the like. Among these, sodium salt, potassium salt, calcium salt, magnesium salt and ammonium salt are preferred, and sodium salt and potassium salt are particularly preferred.

Specific examples of the salt derived from a pharmaceutically acceptable non-toxic organic base include salts of primary, secondary, and tertiary amines, substituted amines including naturally occurring substituted amines, cyclic amine, and basic ion exchange resin. Examples of organic bases other than the aforementioned specific examples of organic amines and amino acids include isopropylamine, diethylamine, triethylamine, trimethylamine, tripropylamine, ethylenediamine, N,N'-dibenzylethylenediamine, 2-diethylaminoethanol, 2-dimethylaminoethanol, morpholine, N-ethyl-morpholine, piperazine, piperidine, N-ethylpiperidine, betaine, caffeine, choline, glucamine, glucosamine, histidine, hydrabamine, methylglucamine, polyamine resin, procaine, purine, theobromine and the like.

In the present specification, the "pharmaceutically acceptable carrier" includes various organic or inorganic carrier substances conventionally used as preparation materials, and excipients, disintegrants, binders, fluidizers, lubricants, and the like in solid preparations; solvents, solubilizing agents, suspending agents, isotonicity agents, buffering agents, soothing agents, and the like in liquid preparations; and bases, emulsifiers, wetting agents, stabilizers, stabilizing agents, dispersing agents, plasticizers, pH adjusters, absorption promoters, gelling agents, antiseptics, fillers, dissolving agents, solubilizing agents, suspending agents, and the like in semisolid preparations can be mentioned. If necessary, additives such as preservatives, antioxidants, colorants, sweetening agents, and the like may also be included.

In the present specification, the "IL-6 and/or IL-1β suppressor" means a medicament that suppresses excessive gene expression or production of IL-6 and/or IL-1β, which are cytokines that play an important role in regulating immune responses and inflammatory responses. The "IL-6 and/or IL-1β suppressor" may be a medicament that suppresses gene expression or production of either IL-6 or IL-1β, and more preferably a medicament that suppresses gene expression or production of both IL-6 and IL-1β (dual inhibitor).

In the present specification, the "circulatory diseases accompanied by inflammation of a cardiovascular system related to IL-6 and/or IL-1β" means circulatory diseases accompanied by inflammation of the cardiovascular system (specifically, pulmonary vascular or myocardium) caused by excessive gene expression or production of IL-6 and/or IL-1β. Specific examples of the circulatory disease include cardiac hypertrophy (e.g., right ventricular hypertrophy), heart failure (e.g., right heart failure), myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease (e.g., mixed connective tissue disease, systemic scleroderma), pulmonary arterial hypertension associated with portal hypertension, large vessel vasculitis (e.g., Takayasu's arteritis, giant cell arteritis), aortic aneurysm, artery dissociation, and the like.

In the present specification, the "cardiovascular remodeling" means restructuring of ventricular structure and vascular structure caused by various loads under physiological or pathological environment, and is called ventricular remodeling and vascular remodeling, respectively. While remodeling of these is a purposeful adaptive response for the body at a certain point in time, it is known to become an important factor in the progression of pathological conditions in the long term.

In the present specification, the "cardiovascular remodeling suppressor" is a medicament for improving cardiovascular remodeling by delaying the progression of cardiovascular remodeling or preventing the progression to irreversible pathological remodeling.

In the present specification, the "pulmonary artery muscularization suppressor" means a drug for suppressing the thickening of the pulmonary artery media and adventitia and the muscular arterialization of pulmonary microvessels, which are caused by the proliferation of vascular smooth muscle cells promoted by shear stress, hypoxic environment, inflammation, and the like.

In the present specification, the "pharmaceutically effective amount" means the dose of the compound (I) of the present invention or a pharmaceutically acceptable salt thereof to be orally or parenterally (topical, rectal, intravenous administration, intramuscular, subcutaneous, etc.) administered to a mammal.

In the present specification, the "mammal" is not particularly limited and includes, for example, human, or mammal other than human (e.g., mouse, rat, hamster, guinea pig, rabbit, cat, dog, swine, bovine, horse, sheep, monkey, etc.).

In the present specification, the "prophylaxis" means to inhibit, deter, control, slow down or stop the onset of clinical symptoms of a disease and the like in a patient (or subject) who may develop but has not yet developed the disease and the like, or who has concerns about the recurrence of the disease and the like after treatment for the disease and the like.

In the present specification, the "treatment" means the remission, mitigation, or delay in the exacerbation of clinical symptoms of a disease, illness, disorder, etc. (hereinafter referred to as "disease, etc.") in a patient (or subject) who has developed the disease, etc.

### (The compound of the present invention)

The compound of the present invention is a compound represented by the following formula (I): 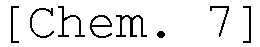 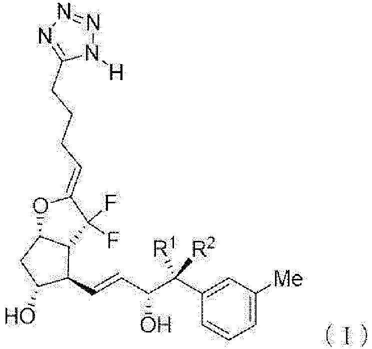 wherein R¹ and R² are each independently a hydrogen atom or a methyl group.

Preferred combinations of R¹ and R² in the compound of the present invention represented by the aforementioned formula (I) are as follows.

R¹ is a hydrogen atom and R² is a hydrogen atom.

R¹ is a methyl group and R² is a hydrogen atom.

R¹ is a hydrogen atom and R² is a methyl group.

R¹ is a methyl group and R² is a methyl group.

Among the above-mentioned combinations, preferred combinations are as follows from the aspect of the high action to suppress excessive gene expression or production of IL-6 and/or IL-1β.

R¹ is a methyl group and R² is a hydrogen atom.

R¹ is a hydrogen atom and R² is a methyl group.

Furthermore, a particularly preferred compound of the present invention is 4-[(Z)-(1S,5R,6R,7R)-6-[(1E,3R,4R)-3-hydroxy-4-(m-tolyl)-1-pentenyl]-7-hydroxy-2-oxa-4,4-difluoro-bicyclo[3.3.0]octan-3-ylidene]-1-(tetrazol-5-yl)butane (at times referred to as "KAG-308" in the present specification).

The compound of the present invention has an asymmetric carbon in the structure thereof and thus exists in various stereoisomers and optical isomers. The present invention includes all of these stereoisomers, optical isomers, and mixtures thereof.

In addition, the compound of the present invention or a pharmaceutically acceptable salt thereof also includes solvates thereof (e.g., hydrates, ethanol solvates, etc.).

### (Production method of the compound of the present invention)

The compound of the present invention or a pharmaceutically acceptable salt thereof can be produced by a known method, for example, the method described in Patent Literature 1 (WO 2010/029925) or Patent Literature 2 (WO 2011/111714) related to the invention of the present inventors.

### (Characteristics of the compound of the present invention)

The compound of the present invention or a pharmaceutically acceptable salt thereof is a prostaglandin (PG) derivative that is less susceptible to metabolism in vivo and has improved stability. Since the carboxy group of the PG skeleton is converted to a tetrazole group, it is less susceptible to metabolism by β-oxidation, which is known as a generally metabolic pathway for fatty acids such as PG and the like. For this reason, compared to compounds having a carboxy group in the PG skeleton, it has a long half-life in the blood and can maintain an effective blood concentration for a long period of time. In addition, such improved metabolic stability can improve the bioavailability of the drug.

The compound of the present invention or a pharmaceutically acceptable salt thereof exhibits an action as an IL-6 and/or IL-1β suppressor. Preferred examples of the compound having such an action are the same as the aforementioned preferred embodiments of the compound of the present invention.

### (Medicaments containing the compound of the present invention or a pharmaceutically acceptable salt thereof (or IL-6 and/or IL-1β suppressor consisting of the compound of the present invention or a pharmaceutically acceptable salt thereof) as an active ingredient)

The medicament of the present invention may be a medicament consisting only of the compound of the present invention or a pharmaceutically acceptable salt thereof, or a pharmaceutical composition containing a pharmaceutically acceptable carrier, and other treatment components (preferably, one or two or more kinds of vasodilators) as necessary.

The dosage of the medicament (or pharmaceutical composition) of the present invention when administered to a mammal (patient or subject) including humans varies depending on the subject of administration, disease, symptoms, dosage form, administration route, and the like. For example, in the case of humans, when administered to an adult patient (body weight about 60 kg), the daily dosage varies depending on the age, body weight, pathological condition, and severity of the patient. When converted to the compound of the present invention as the active ingredient, it is generally 0.0001 to 10 mg, preferably 0.01 to 1 mg, which is desirably administered in one or several divided doses. For example, in oral administration, 0.001 to 3 mg is preferred, 0.02 to 0.5 mg is particularly preferred. In intravenous administration, 0.0001 to 1 mg is preferred, 0.001 to 0.2 mg is particularly preferred. The dosage can be increased or decreased as appropriate depending on the disease or pathological condition. In addition, in the case of an injection, a desirable dosage form may be administration by continuous infusion.

When used as a medicament (or pharmaceutical composition), it can be administered to the body by oral administration or parenteral administration (e.g., intravascular (intravenous, intraarterial) administration, subcutaneous administration, intrarectal administration etc.). Examples of the dosage form include oral dosage forms such as tablet, capsule, syrup and the like, and parenteral dosage forms such as liquid injections of solution, emulsion, suspension or the like, infusion, suppository, nasal drop, adhesive preparation, inhalant, and the like. Among these, oral administration is preferred, and oral administration in two or more divided doses per day is particularly preferred.

The pharmaceutical composition of the present invention (hereinafter at times referred to as "the preparation of the present invention") is produced by appropriately mixing the compound of the present invention or a pharmaceutically acceptable salt thereof with at least one or more kinds of pharmaceutically acceptable carriers, and the like, in an appropriate amount, according to a method known in the technical field of pharmaceutical preparations. For example, when the preparation is powder, granule, tablet, or the like, it can be produced using any pharmaceutically acceptable carrier (e.g., excipient, lubricant, disintegrant, binder, etc.) suitable for producing solid preparations. While the content of the compound of the present invention or a pharmaceutically acceptable salt thereof varies depending on the dosage form, dosage, and the like, it is, for example, 0.1 wt% to 100 wt% of the entire preparation or medicament.

A pharmaceutically acceptable carrier that can be used when the preparation of the present invention is a solid preparation is not particularly limited. Examples of the "excipient" include lactose, sucrose, D-mannitol, D-sorbitol, cornstarch, dextrin, microcrystalline cellulose, crystalline cellulose, carmellose, carmellose calcium, sodium carboxymethyl starch, low-substituted hydroxypropylcellulose, calcium carbonate, sodium carbonate, calcium phosphate, sodium phosphate, alginic acid, gum arabic and the like; examples of the "lubricant" include magnesium stearate, calcium stearate, stearic acid, talc and the like; examples of the "disintegrant" include carmellose, calcium carmellose, sodium carmellose, sodium carboxymethyl starch, sodium croscarmellose, crospovidone, low-substituted hydroxypropylcellulose, hydroxypropylmethylcellulose, crystalline cellulose and the like; and examples of the "binder" include hydroxypropylcellulose, hydroxypropylmethylcellulose, povidone, crystalline cellulose, sucrose, dextrin, starch, gelatin, sodium carmellose, gum arabic and the like. The solid preparation may be uncoated or may be coated by a known technique in order to delay disintegration and absorption in the gastrointestinal tract and thereby provide a sustained action over a longer period. For example, a time-delayed material, such as glyceryl monostearate or glyceryl distearate, may be used.

The preparation of the present invention may be a hard gelatin capsule in which the compound of the present invention is mixed with an inactive solid diluent, for example, calcium carbonate, calcium phosphate, or kaolin, or may be provided as a soft gelatin capsule in which the compound is mixed with a water-miscible solvent (e.g., propylene glycol, polyethylene glycol, ethanol, etc.) or an oil medium (e.g., peanut oil, liquid paraffin, olive oil, etc.).

When the preparation of the present invention is a syrup or liquid, for example, it can be produced by appropriately selecting stabilizer, suspending agent, corrigent, aromatic, and the like. When the medicament of the present invention is an injection, it can be produced by dissolving an active ingredient in distilled water for injection, together with a pH adjuster such as hydrochloric acid, sodium hydroxide, lactose, sodium lactate, acetic acid, sodium monohydrogen phosphate, sodium dihydrogen phosphate, or the like, and an isotonicity agent such as sodium chloride, glucose, or the like, and aseptically preparing same. Alternatively, the preparation may be prepared using an inactive non-aqueous diluent such as propylene glycol, polyethylene glycol, olive oil, ethanol, polysorbate 80, or the like, or may be prepared as an injection for dissolution when in use by further adding mannitol, dextrin, cyclodextrin, gelatin, or the like and freeze-drying the mixture in a vacuum. Also, in order to improve stabilizing performance and lesion reachability, a liposome preparation or a lipid emulsion may be prepared by a known method and used as an injection.

As other embodiments of the preparation of the present invention, an intrarectal administration preparation using a suppository base such as cacao butter, fatty acid triglyceride, fatty acid diglyceride, fatty acid monoglyceride, polyethylene glycol and the like; an intrarectal injecting ointment prepared to have a suitable viscosity by using a water-soluble base such as polyethylene glycol, polypropylene glycol, glycerol, glycerol gelatin and the like, an oily base such as white petrolatum, hard fat, paraffin, liquid paraffin, plastibase, lanolin, purified lanolin and the like; and the like can also be mentioned.

The compound of the present invention or a pharmaceutically acceptable salt thereof can be topically administered to the skin or mucosa, i.e., transdermally or transmucosally. As general dosage forms for this purpose, gel, hydrogel, lotion, solution, cream, ointment, dusting powder, dressing, foam preparation, film, skin patch, oblate, implant, sponge, fiber, bandage, microemulsion and the like can be mentioned. As a pharmaceutically acceptable carrier that can be used for this purpose, alcohol, water, mineral oil, liquid paraffin, white petrolatum, glycerol, polyethylene glycol, propylene glycol, and the like can be mentioned.

For use in any of the aforementioned dosage forms, the compound of the present invention may be mixed with a soluble polymeric unit such as cyclodextrin and an appropriate derivative thereof or a polyethylene glycol-containing polymer in order to improve the solubility, dissolution rate, bioavailability and stability thereof. For example, a drug-cyclodextrin complex and the like have been confirmed to be generally useful for the majority of dosage forms and administration routes. Both inclusion and non-inclusion complexes can be used. As another method of direct complexation with the compound of the present invention, the cyclodextrin may also be used as an auxiliary additive, i.e. as a carrier, excipient, or solubilizer. For these purposes. α-, β- and γ-cyclodextrins and the like are generally used.

The compound of the present invention or a pharmaceutically acceptable salt thereof can be used in combination with other drugs (concomitant drugs) as long as the efficacy thereof is not impaired.

Other drugs (concomitant drugs) preferable for use in combination with the compound of the present invention or a pharmaceutically acceptable salt thereof are not particularly limited. For example, one or more kinds of known medicaments conventionally used for the treatment of a circulatory disease selected from the group consisting of hypertension, heart failure, pulmonary arterial hypertension, thrombosis, arrhythmia, ischemic heart disease, cardiomyopathy, arterial disease, myocarditis, and arteriosclerosis, an autoimmune disease, or a respiratory disease can be preferably used. Specifically, for example, vasodilators such as prostacyclin derivatives (epoprostenol, beraprost, iloprost, treprostinil, selexipag, etc.), endothelin receptor antagonists (bosentan, ambrisentan, macitentan, etc.), phosphodiesterase 5 (PDE5) inhibitors (sildenafil, tadalafil, etc.), guanylate cyclase stimulators (riociguat, etc.), and the like; steroids such as predonisolone, methylprednisolone, dexamethasone, and the like; immunosuppressants such as MTX, azathioprine, cyclophosphamide, mycophenolate mofetil, tacrolimus, cyclosporine, and the like; biological preparations such as tocilizumab, TNF inhibitor, and the like; anti-platelet drugs such as aspirin and the like; and the like can be mentioned. Among these, vasodilators are particularly preferred.

The concomitant drugs that complement and/or enhance the therapeutic effects of the compound of the present invention or a pharmaceutically acceptable salt thereof include those that have not been discovered so far but will be discovered in the future, based on the above-mentioned mechanism (i.e., the mechanism of suppressing excessive gene expression or production of IL-6 and/or IL-1β).

When a concomitant drug is used, the administration period is not limited, and it may be administered to an administration subject at the same time as or at a different time from (i.e., with a time lag) the administration of the medicament of the present invention. In the case of administration with a time lag, the medicament of the present invention may be administered first and the concomitant drug may be administered later, or the concomitant drug may be administered first and the medicament of the present invention may be administered later. The administration methods thereof may be the same or different. In addition, the compound of the present invention or a pharmaceutical acceptable salt thereof and the concomitant drug may be administered in combination as a single preparation (combination drug). Pharmaceutically acceptable carriers that may be used in the production of the combination drug are the same as those used in the pharmaceutical composition of the present invention described above.

The dose of the concomitant drug can be appropriately selected based on the dosage generally used in clinical practice. The mixing ratio of the compound of the present invention or a pharmaceutically acceptable salt and the concomitant drug can be appropriately selected according to the administration subject (age, body weight, general health status, gender, the disease state, etc. of the subject), administration route, the type of disease, the kind of concomitant drug, and the like.

The mass ratio of the compound of the present invention or a pharmaceutically acceptable salt thereof and the concomitant drug is not particularly limited.

The medicament or pharmaceutical composition of the present invention may be provided in the form of a kit, together with an instruction manual on the administration method and the like. The medicament contained in the kit is supplied in a container made of a material that effectively maintains the activity of the constituent components of the medicament or pharmaceutical composition for a long period of time, does not adsorb to the inner side of the container, and does not alter the constituent components. For example, a sealed glass ampoule may contain a buffer or the like encapsulated in the presence of a neutral, inert gas such as nitrogen gas and the like.

The kit may also be accompanied by an instruction manual. The use explanation of the kit may be printed on paper or stored on an electromagnetically readable medium such as a CD-ROM or DVD-ROM and supplied to the user.

### (Use of medicament containing the compound of the present invention or a pharmaceutically acceptable salt thereof as active ingredient)

A medicament containing an IL-6 and/or IL-1β suppressor consisting of the compound of the present invention or a pharmaceutically acceptable salt thereof as an active ingredient can be applied to the treatment of diseases related to IL-6 and/or IL-1β, particularly, circulatory diseases with inflammation of a cardiovascular system induced by excessive gene expression or production of IL-6 and/or IL-1β. The medicament of the present invention is particularly useful for circulatory diseases accompanied by pulmonary vascular or myocardial inflammation.

The medicament of the present invention shows a potent cardiovascular remodeling suppressive action, a right ventricular systolic pressure reduction action, and an anti-inflammatory action, and therefore, it is useful as a therapeutic drug for cardiac hypertrophy (particularly, right ventricular hypertrophy) among the circulatory diseases.

The medicament of the present invention shows a therapeutic effect not only on cardiac hypertrophy but also pulmonary hypertension, and also has a potent cardiovascular remodeling suppressive action, a right ventricular systolic pressure reduction action, and an anti-inflammatory action. Therefore, it is also useful as a therapeutic drug for heart failure (particularly, right heart failure).

The medicament of the present invention has a potent cardiovascular remodeling suppressive action, particularly pulmonary artery muscularization suppressive action, a right ventricular systolic pressure reduction action, an anti-inflammatory action, and an immunosuppressive action, and therefore, it is useful for pulmonary hypertension among the circulatory diseases. The medicament of the present invention is particularly useful as a therapeutic drug for a disease selected from the group consisting of pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease (e.g., mixed connective tissue disease, systemic scleroderma), and pulmonary arterial hypertension associated with portal hypertension.

The medicament of the present invention has a potent cardiovascular remodeling suppressive action, particularly an artery muscularization suppressive action, an anti-inflammatory action, and an immunosuppressive action. Therefore, it is particularly useful as a therapeutic drug for a disease selected from the group consisting of large vessel vasculitis (e.g., Takayasu's arteritis, giant cell arteritis), aortic aneurysm, and artery dissociation, among the circulatory diseases.

### [Example]

The present invention is specifically explained in the following by referring to Examples; however, the present invention is not limited to the Examples.

### [Example 1]

### (Method)

According to the method described in Abe K, Circ Res, 94: 385-393, 2004, a monocrotaline (MCT)-induced pulmonary hypertension rat model was prepared. Monocrotaline (manufactured by Sigma Ltd. Aldrich) was subcutaneously injected once at a dose of 60 mg/kg to 5- to 6-week-old (body weight 150-200 g) male Sprague Dawley rats (SD rat). The compound of the present invention (KAG-308) was orally administered using a gavage needle at a dose of 1 mg/kg (2 mg/kg/day) twice daily from the day of subcutaneous injection of monocrotaline until the day before measurement ("MCT+KAG-308" in Fig. 1). The same amount of solvent (water for injection) was administered for the same period to the pulmonary hypertension rat model not treated with KAG-308 ("MCT" in Fig. 1). The normal control group, which was not administered monocrotaline and KAG-308, was of the same age ("Normal" in Fig. 1). Three weeks after monocrotaline injection, right ventricular systolic pressure, systemic arterial pressure, heart rate number, and right-to-left ventricular weight ratio (right ventricular hypertrophy) were measured. Under isoflurane inhalation anesthesia, right ventricular systolic pressure was measured by the right heart catheterization method. Using a conductance catheter, systemic arterial pressure and heart rate were measured from the carotid artery. After the measurement, the rats were euthanized with a lethal dose of intravenous pentobarbital, and the hearts were excised. The weight of the left ventricle (LV) including the ventricular septum (Sep) relative to the right ventricle (RV) was measured, and the ratio was used as an index of right ventricular hypertrophy.

### (Results)

### (1) Effect on right ventricular systolic pressure and right ventricular hypertrophy

As shown in the left figure in Fig. 2, KAG-308 significantly suppressed an increase in right ventricular systolic pressure (RVSP) observed in rats with monocrotaline-induced pulmonary hypertension.

As shown in the right figure in Fig. 2, KAG-308 significantly suppressed an increase in the weight ratio of the left ventricle (LV), including the ventricular septum (Sep), to the right ventricle (RV) (RV/LV + Sep) observed in rats with monocrotaline-induced pulmonary hypertension.

### (2) Effect on left ventricular systolic pressure and heart rate

As shown in Fig. 3, there were no significant differences in left ventricular systolic pressure and heart rate among any of the groups.

From the above results, it was confirmed that KAG-308 significantly suppresses an increase in right ventricular systolic pressure and right ventricular hypertrophy without affecting left ventricular systolic pressure and heart rate.

### [Example 2] (Histopathological analysis)

To examine the effect of 3-week administration of KAG-308 (2 mg/kg/day) in the monocrotaline-induced pulmonary hypertension rat model shown in Example 1, histopathological analysis was performed.

### (Method)

Lungs taken from the rats used in the test in Example 1 were expanded by applying 20 cm H₂O pressure, and fixed overnight with 10% formalin. Then, the tissue sections embedded in paraffin and cut to 5 µm were subjected to Elastica Van Gieson (EVG) staining and immunohistological staining. The analysis was performed using Graph Pad Prism 8 to calculate the mean medial wall thickness of the pulmonary artery and the muscularization of small pulmonary arterioles.

### (Results)

### (1) Effect on medial wall thickness of pulmonary artery

Pulmonary arteries with an outer diameter of 50 to 100 µm were analyzed, and the mean medial wall thickness was measured in a blind manner (mean medial wall thickness = (outer diameter -inner diameter)/outer diameter). The results are shown in Fig. 4.

According to Fig. 4, the average medial wall thickness increased in the monocrotaline administration group, but the increase in the average medial wall thickness was significantly suppressed by administration of KAG-308. These results confirmed the suppressive effect of KAG-308 on smooth muscle proliferation and thickening due to pulmonary artery remodeling.

### (2) Suppressive effect on muscularization of small pulmonary arteries

Muscularization of small pulmonary arteries with an outer diameter of 50 µm or less was analyzed by immunostaining with anti-α-SMA (smooth muscular actin) antibody. According to the percentage of smooth muscle in the total circumference of the small pulmonary artery, they were defined as Non muscular (<25%), Partially muscular (25 - 75%), and Totally muscular (>75%), and at least 70 small pulmonary arteries of each specimen were evaluated in a blinded method. The results are shown in Fig. 5.

According to Fig. 5, the non-muscular percentage increased and the totally muscular percentage decreased in the KAG-308 administration group. These results confirmed that administration of KAG-308 had a clear suppressive effect on the muscularization of small pulmonary arteries.

### (3) Inflammatory macrophage infiltration suppressive effect

Macrophages were measured by immunostaining using anti-CD68 antibody. The device used was a laser scanning microscope FV1200 IX83 (OLYMPUS), and observation was performed at a magnification of 400x, and CD68-positive cells were counted in 15 randomly selected fields mainly of small blood vessels. The results are shown in Fig. 6.

According to Fig. 6, in the monocrotaline-induced pulmonary hypertension rats, infiltration of inflammatory CD68-positive macrophages increased, but the increase was significantly suppressed by the administration of KAG-308. The suppressive effect on the infiltration of inflammatory macrophages was confirmed.

### [Example 3] (Quantitative RT-PCR analysis)

According to the method described in Abe, K. et al. Cardiovasc Res. 2016, 111, 16, to examine the effect of KAG-308 (2 mg/kg/day) in the monocrotaline-induced pulmonary hypertension rat model shown in Example 1, mRNA quantification analysis of IL-6 and IL-1β was performed by the PCR method.

### (Method)

Lungs taken from the rats used in the test in Example 1 were disrupted, and total RNA was extracted from the whole lung by using a Qiagen RNeasy mini kit. ReverTra Ace qPCR Kit (TOYOBO CO., LTD.) and SYBR Premix Ex Taq (Takara Bio Inc.) were respectively used for reverse transcription and amplification. Using TB Green Premix Ex TaqII (Takara Bio Inc.), 7500 real-time PCR system (Applied biosystems), the analysis was performed. The primers used are shown in the following.

### Primer sequence

IL-6 F: AGCGATGATGCACTGTCAGAA
IL-6 R: AACGGAACTCCAGAAGACCAG
IL-1β F: CCCTGAACTCAACTGTGAAATAGCA
IL-1β R: CCCAAGTCAAGGGCTTGGAA

### (Results)

According to Fig. 7, in monocrotaline administered rats, it was confirmed that the mRNA expression level of IL-6, which is an inflammatory cytokine, increased 60-fold or more (left figure), and the mRNA expression level of IL-1β increased 2-fold or more (right figure). On the other hand, in rats administered with KAG-308 (2 mg/kg/day), the increase in the mRNA expression levels of IL-6 and IL-1β was significantly suppressed. In particular, the increase in the mRNA expression level of IL-6 was suppressed by 89%.

From the above results, it was confirmed that the compound of the present invention significantly suppresses right ventricular systolic pressure, right ventricular hypertrophy, and pulmonary artery muscularization as compared with non-treated monocrotaline-induced pulmonary hypertension group, without changing the heart rate and left ventricular systolic pressure. As the mechanism thereof, the prophylactic and therapeutic effect on pulmonary hypertension and the right ventricular hypertrophy suppressive effect, afforded by the suppression of cardiovascular remodeling due to the suppressive effect on excessive gene expression or production of IL-6 and/or IL-1β, were confirmed. Therefore, it was shown that the compound of the present invention is useful for the treatment of pulmonary hypertension and cardiac hypertrophy via the suppression of IL-6 and/or IL-1β.

### Formulation Example 1: production of capsule

| | |
|---|---|
| 1) compound of the present invention (KAG-308) | 0.05 mg |
| 2) microcrystalline cellulose | 20 mg |
| 3) lactose | 29 mg |
| 4) magnesium stearate | 1 mg |

| | |
|---|---|
| 1), 2), 3) and 4) are mixed and filled in a gelatin capsule. | |

### Formulation Example 2: production of tablet

| | |
|---|---|
| 1) compound of the present invention (KAG-308) | 30 mg |
| 2) lactose | 70.0 g |
| 3) cornstarch | 50.0 g |
| 4) soluble starch | 7.0 g |
| 5) magnesium stearate | 3.0 g |

The compound of the present invention (30 mg) and magnesium stearate (3.0 g) are granulated with an aqueous soluble starch solution (70 mL) (7.0 g as soluble starch), dried, mixed with lactose (70.0 g) and cornstarch (50.0 g) (lactose, cornstarch, soluble starch, and magnesium stearate are all compliant with the 14th edition of the Japanese Pharmacopoeia). The mixture is compressed to obtain tablets.

### [Industrial Applicability]

The compound of the present invention or a pharmaceutically acceptable salt thereof is useful as an IL-6 and/or IL-1β suppressor. According to the present invention, moreover, a medicament for the treatment of circulatory diseases accompanied by inflammation of a cardiovascular system related to IL-6 and/or IL-1β, in particular, a disease selected from the group consisting of cardiac hypertrophy (e.g., right ventricular hypertrophy), heart failure (e.g., right heart failure), myocarditis, cardiomyopathy, pulmonary hypertension accompanied by right heart failure, pulmonary hypertension associated with left heart disease, pulmonary hypertension associated with lung disease and/or hypoxemia, chronic thromboembolic pulmonary hypertension, pulmonary arterial hypertension associated with connective tissue disease (e.g., mixed connective tissue disease, systemic scleroderma), pulmonary arterial hypertension associated with portal hypertension, large vessel vasculitis (e.g., Takayasu's arteritis, giant cell arteritis), aortic aneurysm, and artery dissociation, which contains, as an active ingredient, an IL-6 and/or IL-1β suppressor consisting of the compound of the present invention or a pharmaceutically acceptable salt thereof, can be provided.

This application is based on a patent application No. 2022-080531 filed in Japan (filing date: May 16, 2022), the contents of which are incorporated in full herein.

## Claims

1. An IL-6 and/or IL-1β suppressor consisting of a compound represented by the formula (I): 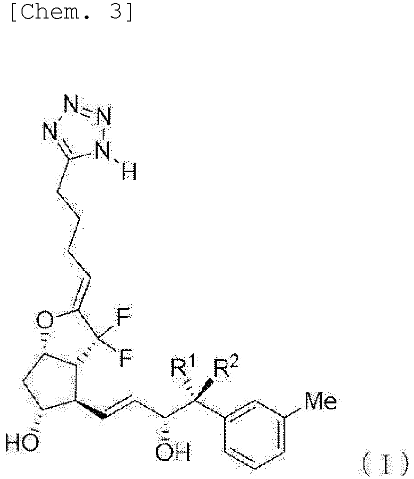 wherein R¹ and R² are each independently a hydrogen atom or a methyl group, or a pharmaceutically acceptable salt thereof.

2. The suppressor according to claim 1, wherein the aforementioned compound represented by the formula (I) is 4-[(Z)-(1S,5R,6R,7R)-6-[(1E,3R,4R)-3-hydroxy-4-(m-tolyl)-1-pentenyl]-7-hydroxy-2-oxa-4,4-difluoro-bicyclo[3.3.0]octan-3-ylidene]-1-(tetrazol-5-yl)butane.

3. The suppressor according to claim 1 or 2, which is an IL-6 and IL-1β suppressor.

4. A medicament for the treatment of a circulatory disease accompanied by inflammation of a cardiovascular system related to IL-6 and/or IL-1β, comprising the suppressor according to any one of claims 1 to 3 as an active ingredient.

5. The medicament according to claim 4, wherein the cardiovascular system is a pulmonary blood vessel.

6. The medicament according to claim 4, wherein the cardiovascular system is the myocardium.

7. The medicament according to any one of claims 4 to 6, wherein the circulatory disease is selected from the group consisting of cardiac hypertrophy, heart failure, myocarditis, and cardiomyopathy.

8. The medicament according to claim 7, wherein the circulatory disease is cardiac hypertrophy.

9. The medicament according to claim 7, wherein the circulatory disease is heart failure.

10. The medicament according to claim 9, wherein the heart failure is right heart failure.

11. The medicament according to claim 4, wherein the circulatory disease is pulmonary hypertension accompanied by right heart failure.

12. The medicament according to claim 4, wherein the circulatory disease is pulmonary hypertension associated with a left heart disease.

13. The medicament according to claim 4, wherein the circulatory disease is pulmonary hypertension associated with lung disease and/or hypoxemia.

14. The medicament according to claim 4, wherein the circulatory disease is chronic thromboembolic pulmonary hypertension.

15. The medicament according to claim 4, wherein the circulatory disease is pulmonary arterial hypertension associated with a connective tissue disease.

16. The medicament according to claim 15, wherein the connective tissue disease is a mixed connective tissue disease or systemic scleroderma.

17. The medicament according to claim 4, wherein the circulatory disease is pulmonary arterial hypertension associated with portal hypertension.

18. The medicament according to claim 4, wherein the circulatory disease is selected from the group consisting of large vessel vasculitis, aortic aneurysm, and artery dissociation.

19. The medicament according to any one of claims 4 to 18, which is for oral administration.

20. The medicament according to claim 19, which is orally administered in two or more divided doses per day.

21. The medicament according to any one of claims 4 to 20, which is administered in combination with one or two or more kinds of other drugs.

22. The medicament according to claim 21, wherein the medicament according to claim 4 and one or two or more kinds of other drugs are contained separately as active ingredients of different preparations and are administered simultaneously or at different times.

23. The medicament according to claim 21, wherein the medicament according to claim 4 and one or two or more kinds of other drugs are contained in a single preparation.

24. The medicament according to any one of claims 21 to 23, wherein the other drug is a vasodilator.

25. A pharmaceutical composition comprising the suppressor according to any one of claims 1 to 3, and a pharmaceutically acceptable carrier.

26. A cardiovascular remodeling suppressor comprising the suppressor according to any one of claims 1 to 3 as an active ingredient.

27. A pulmonary artery muscularization suppressor comprising the suppressor according to any one of claims 1 to 3 as an active ingredient.
